# EUROPÄISCHE PATENTSCHRIFT

(11) **EP 1 335 715 B1**
(45) Veröffentlichungstag und Bekanntmachung des Hinweises auf die Patenterteilung: **11.05.2005**
(21) Anmeldenummer: 01993464.5
(22) Anmeldetag: 31.10.2001
(51) Int. Cl.: A61K 9/70

(54) **HAUTFREUNDLICHES WIRKSTOFFPFLASTER ZUR TRANSDERMALEN VERABREICHUNG HYPERÄMISIERENDER WIRKSTOFFE**
SKIN-FRIENDLY AND DRUG-CONTAINING PATCHES FOR TRANSDERMAL ADMINISTRATION OF HYPEREMIC ACTIVE INGREDIENTS
TIMBRE RENFERMANT UNE SUBSTANCE ACTIVE, DOUX POUR LA PEAU ET PERMETTANT L'ADMINISTRATION TRANSDERMIQUE DE SUBSTANCES ACTIVES HYPEREMIANTES

(30) Priorität: 11.11.2000 DE 10056009
(43) Veröffentlichungstag der Anmeldung: 20.08.2003
(73) Patentinhaber: Beiersdorf AG, 20245 Hamburg (DE)
(72) Erfinder: RADLOFF, Detlev, 22769 Hamburg (DE); WASNER, Matthias, 21029 Hamburg (DE)
(86) Internationale Anmeldenummer: PCT/EP2001/012605
(87) Internationale Veröffentlichungsnummer: WO 2002/038137

(56) Entgegenhaltungen:
- EP-A- 0 664 119
- EP-A- 0 846 463
- EP-A- 1 120 115
- WO-A-00/45797
- WO-A-98/54268

## Beschreibung

Die Erfindung betrifft ein arzneistoffhaltiges Pflastersystem auf der Basis synthetischer Kautschuke zur Abgabe von mindestens einem hyperämisierenden Wirkstoff zur Aufnahme durch die Haut über einen Zeitraum von bis zu 24 h sowie ein Verfahren zu seiner Herstellung.

Transdermal Therapeutische Systeme (TTS) zur Abgabe von Wirkstoffen durch die Haut sind seit langer Zeit bekannt. Die topische Applikation von Arzneimittel über wirkstoffhaltige Pflastersysteme bietet zwei Hauptvorteile: Erstens wird durch diese Darreichungsform eine Freisetzungskinetik des Wirkstoffes erster Ordnung realisiert, wodurch über einen sehr langen Zeitraum ein konstanter Wirkstoffspiegel im Organismus aufrechterhalten werden kann. Zweitens werden über den Aufnahmeweg durch die Haut der Magen-Darm-Trakt sowie die erste Leberpassage vermieden. Dadurch können ausgewählte Arzneistoffe in einer geringen Dosierung wirkungsvoll verabreicht werden. Dies ist insbesondere dann von Vorteil, wenn eine lokale Wirkung des Arzneistoffes unter Umgehung einer systemischen Wirkung erwünscht ist. Dies ist zum Beispiel bei der Behandlung rheumatischer Gelenkbeschwerden oder Muskelentzündungen der Fall.

Eine in der Fachliteratur gut beschriebene Ausführungsform solcher transdermalen Systeme stellen Matrixsysteme oder monolitische Systeme dar, in denen der Arzneistoff direkt in den druckempfindlichen Haftklebstoff eingearbeitet wird. Eine solche haftklebrige, wirkstoffhaltige Matrix ist im anwendungsfertigen Produkt auf der einen Seite mit einem für den Wirkstoff undurchlässigen Träger ausgestattet, auf der gegenüberliegenden Seite befindet sich eine mit einer Trennschicht ausgestatten Trägerfolie, die vor der Applikation auf die Haut entfernt wird (kleben&dichten, Nr.42, 1998, S. 26 bis 30).

Eine Grundanforderung an ein TTS ist ein sehr gutes Haftvermögen auf Haut, das über den gesamten Zeitraum der beabsichtigten Wirkstoffdosierung aufrechterhalten bleiben muß. Eine häufig beobachtete Nebenwirkung ist jedoch das Auftreten von Hautirritationen, die besonders bei längerer oder wiederholter Applikation eines TTS an einer gleichbleibenden Körperregion auftreten. Sie werden hauptsächlich durch die Inhaltsstoffe der haftklebrigen Matrix verursacht. Auch ein schmerzhaftes Wiederablösen des wirkstoffhaltigen Pflasters nach längerer Tragezeit wird häufig beobachtet.

Wiederholte und lange andauernde Anwendungen von haftklebrigen Systemen an immer gleichen Regionen des menschlichen Körpers sind vor allem im Bereich der Stoma-Versorgung anzutreffen. Hier werden seit langer Zeit und mit großem Erfolg Hydrokolloide als Haftklebstoff eingesetzt Diese bestehen prinzipiell aus einer hydrophoben haftklebrigen Polymermatrix auf der Basis synthetischer Kautschuke, in der in dieser Matrix unlösliche hydrophile Füllstoffe auf der Basis von zum Beispiel Alginaten, Cellulose oder Pektinen dispers verteilt vorliegen.
Bei der Entwicklung von Hydrokolloiden zur Stoma-Versorgung stehen jedoch die Hafteigenschaften auf feuchter Haut und die Fähigkeit der Flüssigkeitsaufnahme im Vordergrund.

Bereits 1967 wird in US 3,339,546 ein Hydrokolloid auf der Basis von Polyisobutylenen zur Anwendung im Mundraum beschrieben. Ein großer Nachteil der frühen Systeme besteht in der mangelhaften Integrität der Matrices, d.h. in einem Auflösen und Zerfall der haftklebrigen Matrix bei der Aufnahme größerer Mengen an Flüssigkeit.

Spätere Entwicklungen zielen deshalb auf die Lösung dieses Problems ab, und mehrere Lösungsansätze werden in der Literatur aufgezeigt.

US 4,393,080 beschreibt zum Beispiel ein Hydrokolloid-System auf der Basis von Elastomeren, wobei hochmolekulare hydrophile Füllstoffe eingesetzt werden, die einen Zusammenhalt auch des gequollenen Systems fördern.
Weitere Dokumente beschreiben Lösungsansätze über die Vernetzung der Elastomermatrix, die entweder physikalisch oder chemisch verlaufen kann.

Eine physikalische Vernetzung kann zum Beispiel durch den Einsatz phasenseparierender Blockpolymere auf der Basis von Poly(styrol-b-isopren-b-styrol) (SIS), Poly(styrol-b-isopren-b-styrol) (SBS) oder Poly[styrol-b-(ethylen-stat.-butylen)-b-styrol] (SEBS) erfolgen. Eines der ersten solcher Systeme wird zum Beispiel in der DE 28 22 535 beschrieben.

Eine chemische Vernetzung kann zum Beispiel über Elektronen- oder γ-Bestrahlung der Hydrokolloidmatrix erfolgen. Hierbei wird vorausgesetzt, daß in der haftklebrigen Matrix genügend reaktive Strukturelemente vorhanden sind. Dies kann beispielsweise, wie in US 4,477,325 beschrieben, über die Compoundierung mit einem Ethylen-Vinylacetat Copolymer erfolgen.

Innerhalb der vorgenannten Erfindungen wird zwar das technische Problem der Kohäsivität gequollener Hydrokolloide beschrieben und gelöst, das Problem der Hautreizung durch wiederholte Anwendung wird jedoch nicht adressiert.

Mit möglicherweise auftretenden Hautreizungen beschäftigt sich hingegen WO 98/01167 A1. Hier wird Aloe Vera Extrakt zur Vermeidung entzündlicher Hautveränderungen sowie Infektionen bei der Stoma-Versorgung eingesetzt. Das beschriebene System besteht jedoch lediglich aus einem niedermolekularen Polyisobutylen als Polymergerüst, wodurch das zuvor beschriebene Problem der Kohäsivität der Hydrokolloid-Matrix weiterhin besteht. Zusätzlich werden in der beschriebenen Zusammensetzung Klebharze eingesetzt, deren allergenes Potential bekannt ist.

Informationen bezüglich der Eignung eines solchen Systems zur kontrollierten Abgabe von Arzneistoffen sind weder dieser noch in einer anderen der genannten Schriften enthalten.

Transdermal Therapeutischen Systeme werden in der Regel auf gesunder, intakter Haut appliziert. Gerade hier ist es besonders wichtig, die intakte Haut nicht durch ein Arzneimittel zu reizen oder gar zu schädigen. Eine genügende Kohäsivität ist zusätzlich notwendig, um das wirkstoffhaltige Pflaster nach beendeter Tragedauer rückstandsfrei entfernen zu können.

Polyisobutylene werden seit langer Zeit als Gerüstsubstanz bei der Compoundierung von Haftklebstoffen eingesetzt. Gegenüber anderen bekannten Elastomeren bieten synthetische Polymere auf der Basis von Isobutylen eine Reihe von Vorteilen. Sie sind durch ihre synthetische Herstellung frei von unerwünschten Inhaltsstoffen, durch ihre vollständige Sättigung sind sie sehr oxidationsstabil und sie zeichnen sich je nach Molekulargewicht durch eine einstellbare inhärente Klebrigkeit aus.

Vor allem für die Anwendung auf Haut sind sie deshalb anderen Elastomeren vorzuziehen. So ist zum Beispiel das allergene Potential von Naturkautschuk aufgrund seiner natürlichen Verunreinigungen hinlänglich bekannt. Andere synthetische Kautschuke auf der Basis von Styrol und Isopren beziehungsweise Butadien sind sehr oxidationsempfindlich, wodurch eine aufwendige Additivierung notwendig ist. Ihre hydrierten Derivate auf der Basis von Poly[styrol-b-(ethylen-stat-propylen)-b-styrol] (SEPS) oder Poly[styrol-b-(ethylen-stat.-butylen)-b-styrol] (SEBS) sind zwar oxydationsstabiler, ihnen fehlt es jedoch an inhärenter Klebrigkeit. Aus diesem Grund ist für deren Einsatz als Haftklebstoff zusätzlich die Compoundierung mit Klebharzen unabdingbar, wie beispielsweise in EP 0 651 635 B1 beschrieben. Diese sind in der Regel sehr schlecht definierte Stoffgemische und häufig auf der Basis von Kolophonium. Dadurch ist auch hier ein allergenes Potential nicht auszuschließen.

Der Einsatz von Polyisobutylenen für transdermal Therapeutische Systeme wird in DE 33 47 278 A1 und DE 33 47 277 A1 beschrieben. Hier allerdings immer in der Kombination mit entweder olefinischen Dienkautschuken oder Ktebharzen, die wieder die oben beschriebenen Nachteile aufweisen. Auch der Einsatz amorpher Poly-α-olefine als Zusatz wird beschrieben, ohne jedoch deren Einfluß auf das Gesamtsystem zu erläutern. Der Einsatz von Füllstoffen wird in dieser Beschreibung nicht erwähnt.

Der Einsatz von PIB für transdermale Systeme ohne Zusatz von Klebharzen wird in der US 4,559,222 beschrieben. Notwendig ist hier jedoch der Einsatz sehr großer Mengen Mineralöl, wobei das Verhältnis von Mineralöl zu PIB erfindungsgemäß mindestens 1 beträgt. Weiterhin ist das System auf Wirkstoffe beschränkt, die in Mineralöl mäßig löslich sind. Hierdurch wird zusätzlich ein weichmachender Effekt auf die Matrix ausgeübt. Als Füllstoffe werden mindesten 6 Gew.-% kolloidales Silca eingesetzt. Zu diesem Inhaltsstoff ist bekannt, daß das antiadhäsive Verhalten einer Trennfolie signifikant durch den Einsatz von Silika gestört wird.

WO 96/22083 A1 beschreibt ein System zur transdermalen Verabreichung von Nikotin, das auf der Basis von Polyisobutylen ohne den Zusatz von Mineralöl auskommt. Der notwendige tack der Klebmasse wird hier jedoch über den Einsatz von Klebharzen erreicht. Diese besitzen hinsichtlich Hautverträglichkeit die oben genannten Nachteile. Ein weichmachender Effekt, der das adhäsive Verhalten der Matrix zusätzlich positiv beeinflußt, wird über den in der PIB Matrix löslichen Wirkstoff erreicht Dieses Prinzip der Compoundierung schränkt jedoch die Auswahl der über diese Matrix verabreichbaren Wirkstoffe sehr ein.

Ein System zur transdermalen Verabreichung von Arzneimitteln auf der Basis von Polyisobutylenen, das ohne Verwendung sowohl von Klebharzen als auch Mineralöl auskommt, beschreibt US 5,508,038. Die zentrale Komponente zur Erreichung ausreichender adhäsiver Eigenschaften ist jedoch auch in diesem Fall der Wirkstoff, der erfindungsgemäß ölig und in der unpolaren Matrix gut löslich sein muß. Dies stellt eine sehr starke Einschränkung dar, so daß die Verwendung von hyperämisierenden Wirkstoffen in diesem Zusammenhang auszuschließen ist. Das beschriebene System ist frei von anorganischen beziehungsweise organischen Füllstoffen.

US 5,508,038 adressiert als einzige der genannten Verbindungen das Problem der Hautreizung durch die mögliche Verwendung von Klebharzen.

Der Einsatz amorpher Poly-α-olefine in Haftklebstoffen im allgemeinen ist in der Literatur bekannt US 4,186,258 ist eines der ersten Dokumente, die den Einsatz dieser Substanzklasse im Bereich der Schmelzhaftklebstoffe benennt Ein spezielles Einsatzgebiet wird in dieser Schrift nicht genannt.

US 5,262,216 beschreibt den Einsatz dieser Materialien zusammen mit Klebharzen für Schmelzhaftkleber gezielt für den Einsatzbereich an Selbstklebeetiketten. Besonders ausgelobt wird hier die herausragende UV- und Alterungsbeständigkeit dieser Polymerklasse. Der Einsatzbereich am Menschen wird nicht erwähnt.

WO 98/54268 A1 hingegen beschreibt gezielt den Einsatz amorpher Poly-α-olefine für Anwendungen auf menschlicher Haut. Zusätzlich werden hier amorphe Poly-α-olefine in Kombination mit Füllstoffen verwendet. Allerdings wird speziell der Einsatzbereich der Wundabdeckung beschrieben. Amorphe Poly-α-olefine zeichnen sich in diesem Einsatzgebiet durch ihre hervorragende Strahlenresistenz aus, wodurch erfindungsgemäß gut sterilisierbare Produkte zur Wundversorgung hergestellt werden können. Zusätzlich werden hier amorphe Poly-α-olefine in Kombination mit Klebharzen eingesetzt. Der Aspekt der verminderten Hautreizung wird insgesamt nicht erwähnt. Des weiteren enthält WO 98/54268 A1 keine Anhaltspunkte dafür, das ein solches System zur Abgabe von Arzneistoffen über die menschliche Haut geeignet ist. Das Einsatzgebiet der transdermal therapeutischen Systeme wird in dieser Schrift vollständig ausgeklammert.

Die bisher beschriebenen Systeme zur transdermalen Verabreichung eines Wirkstoffes enthalten keine organischen Füllstoffe. Jedoch sind insbesondere diese Füllstoffe für die Hautfreundlichkeit der zuvor beschriebenen Haftklebstoffe zur Stoma-Versorgung verantwortlich. Durch sie kann während der Tragedauer des Pflasters von der Haut abgegebene Feuchtigkeit sehr gut aufgenommen werden. Das dadurch entstehende Klima unter dem Pflaster führt zur deutlichen Verminderung des Auftretens von Hautmazerationen.

Eine Erfindung eines Wirkstoffpflasters unter Einsatz von in Wasser quellfähigen Füllstoffen beschreibt EP 0 186 019 A1. Hier wird jedoch der positive Einfluß des organischen Füllstoffes auf die Freisetzungsrate des Wirkstoffes beschrieben. Der erfindungsgemäße Füllstoffanteil ist auf 30 Gew.-% begrenzt. Der Aspekt der Verminderung von Hautreizungen wird nicht angesprochen. Zusätzlich werden die beschriebenen Systeme unter Einsatz von Klebharzen realisiert.

Wie oben ausgeführt, ist die Realisierung klebharzfreier und mineralölfreier Haftklebesysteme zur transdermalen Verabreichung von Wirkstoffen auf der Basis von Polyisobutylen nur für solche Arzneistoffe beschrieben, die eine bei Raumtemperatur ölige Konsistenz besitzen beziehungsweise in der Matrix löslich sind. Der Einsatz - hyperämisierender Wirkstoffe wie beispielsweise Nonivamid, natürliches Capsaicin ist daher aufgrund ihrer physikalischen Eigenschaften auf der Basis der bekannten Formulierungen nicht möglich. Keine der beschriebenen klebharzfreien Formulierungen enthält einen hydrophilen Füllstoff.

Zusätzlich ergibt sich folgender Stand der Technik:

EP0846463 legt wirkstoffhaltige Pflaster offen, die aus einem Trägermaterial und einer darauf aufgebrachten Heißschmelzselbstklebemasse bestehen. Die Heißschmelzselbstklebemasse enthält einen hyperämisierenden Wirkstoff (Nonivamid, Cayennepfeffer, Nicotinsäurederivate, Benzylnicotinat, Propylnicotinat), 10-90 Gew. % Blockcopolymere (Phase A aus Polystyrol, Phase B aus Thylen, Propylen, Butylen, Butadien, Isopren), 5-80 Gew. % Klebrigmacher (Öle, Wachse, Harze), weniger als 60 % Weichmacher.

In WO0045797 wird ein transdermales therapeutisches System beschrieben, dessen Matrix einen Wirkstoff, ein Gemisch aus hoch- und niedermolekularem Polyisobutylen sowie aus mikrokristalliner Cellulose, Ethylcellulose, Methylcellulose enthält. Der Wirkstoff kann aus der Gruppe entzündungshemmender und antirheumatischer Verbindungen ausgewählt werden. Weichmacher oder Klebemittel können zugesetzt werden, die aus Mineralöl bestehen.

In EP0664119 besteht die Klebstoffmatrix aus Polyisobutylen, der Füllstoff aus Carboxymethylcellulose. Der Wirkstoff kann ein entzündungshemmendes Analgetikum darstellen, wie Indomethacin, Diclofenac, Salicylsäure, Methylsalicylat, 1-Menthol, Kampfer.

Die druckempfindliche adhesive Zusammensetzung aus WO9854268 ist aus einer Matrix mit amorphen Poly-alpha-Olefinen aufgebaut. Ferner sind Hydrokolloide enthalten, die Cellulosederivate, wie z. B. Carboxymethylcellulose, Methylcellulose, Hydroxypropylmethylcellulose, einschließen.

EP1120115 ist nicht vorveröffentlicht. Es legt ein wirkstoffhaltiges Matrixpflaster zur kontrollierten Abgabe von nichtsteroidalen Antirheumatika an die Haut offen, das aus einer flexiblen Deckschicht und einer wirkstoffhaltigen, wasserunlöslichen haftklebrigen Matrix besteht und dessen Matrix frei von Mineralölen und Klebharzen ist. Es ist aus folgenden Komponenten aufgebaut
a) synthetischen erüstpolymeren auf der Basis von Polyisobutylen zu 25-90 Gew.-%
b) amorphem Poly-alpha olefin zu 5-40 Gew.-%
c) unlöslicher, hydrophiler Füllstoff (durchschnittliche Korngröße von weniger als 100 Mikrometern, 10-60 Gew.-%)
d) Arzneistoff zu 0,001-20 Gew.-%.
Die Arzneistoffe sind der Klasse der nichtsteroidalen Antirheumatika entnommen (Ibuprofen, Ketoprofen, Peroxicam oder Diclofenac).

Ziel der vorliegenden Erfindung ist es, ein Hydrokolloidsystem zur kontrollierten Abgabe eines Arzneistoffes aus der Gruppe der hyperämisierenden Wirkstoffe zu entwickeln, das sowohl eine ausgezeichnete Kohäsivität besitzt als auch aus besonders hautfreundlichen Komponenten aufgebaut ist beziehungsweise unter vollständigem Verzicht hautreizender Komponenten wie zum Beispiel Klebharze realisiert werden kann. Zusätzlich ist ein Herstellprozeß geplant, der unter vollständigem Verzicht von Lösungsmittel auskommt, des weiteren sollen die genannten Nebenwirkungen eines Haftklebstoffes für transdermale Systeme - Hautirritationen und schmerzhaftes Wiederablösen - vermieden werden, was eine deutliche Steigerung des Tragekomforts für den Patienten ergibt.
Es soll ein Matrixsystem auf der Basis von Polyisobutylen zur Verfügung gestellt werden, das ohne herkömmliche Klebharze und Mineralöl in einem lösungsmittelfreien Herstellprozeß realisierbar ist.

Gelöst wird diese Aufgabe durch ein wirkstoffhaltiges Matrixpflaster gemäß dem Hauptanspruch. Gegenstand der Unteransprüche sind vorteilhafte Ausführungsformen des erfindungsgemäßen Pflasters. Des weiteren umfaßt die Erfindung Verfahren zur Herstellung derartiger Pflaster.

Demgemäß betrifft die Erfindung ein wirkstoffhaltiges Matrixpflaster zur kontrollierten Abgabe von hyperämisierenden Wirkstoffen an die Haut, bestehend aus einer flexiblen Deckschicht und einer wirkstoffhaltigen, wasserunlöslichen haftklebrigen Matrix, wobei die haftklebrige Matrix frei von Mineralölen und Klebharzen ist und aufgebaut ist aus
a) synthetischen Gerüstpolymeren auf der Basis von Polyisobutylen zu 25 bis 90 Gew.-%,
b) amorphem Poly-α-olefin zu 5 bis 40 Gew.-%,
c) aus einem unlöslichen, hydrophilen Füllstoff mit einer durchschnittlichen Korngröße von weniger als 100 µm zu 10 bis 60 Gew.-% und
d) dem hyperämisierenden Wirkstoff zu 0,001 bis 20 Gew.-%.

In einer ersten vorteilhaften Ausführungsform des wirkstoffhaltigen Matrixpflasters setzt sich das Polyisobutylen zusammen aus hochmolekularem PIB zu 5 bis 30 Gew.-% und niedermolekularem PIB zu 20 bis 60 Gew.-%.

Eine typischer erfindungsgemäßer Haftklebstoff besteht somit aus folgenden Komponenten:

| | | |
|---|---|---|
| hochmolekulares PIB | 5 - 30 Gew. % | bevorzugt 10 - 20 Gew. % |
| niedermolekulares PIB | 20 - 60 Gew.-% | bevorzugt 30 - 50 Gew. % |
| amorphes Poly-α-olefin | 5 - 30 Gew.-% | bevorzugt 5 - 20 Gew. % |
| hydrophiler Füllstoff | 20 - 60 Gew.-% | bevorzugt 30 - 50 Gew. % |
| Wirkstoff | 0,001 - 20 Gew.-% | bevorzugt 1,0 - 5,0 Gew.-% |

Optional können noch bis zu 20 Gew.-% eines permeationsfördemden Hilfsstoffes zugesetzt werden.

Die genannten Rezepturbestandteile werden dabei wie folgt genauer definiert

### Hochmolekulares PIB:

Polyisobutylen mit einem gewichtsmittleren Molekulargewicht (M_{w}) von 500.000 bis 1.100.000, bevorzugt zwischen 650.000 und 850.000. Solche Polymere sind kommerziell beispielsweise unter den Handelsnamen Oppanol B100 (BASF) oder Vistanex MM-L80 (Exxon) erhältlich.

### Niedrig molekulares PIB:

Polyisobutylen mit einem gewichtsmittleren Molekulargewicht (M_{w}) von 40.000 bis 120.000, bevorzugt zwischen 60.000 und 100.000. Solche Polymere sind kommerziell beispielsweise unter den Handelsnamen Oppanol B15 (BASF) oder Vistanex LMMH (Exxon) erhältlich.

### Amorphes Poly-α-olefin:

Amorphe Copolymere auf der Basis von Ethylen und Propylen, Butylen oder 1-Hexen. Das bevorzugte gewichtsmittlere Molekulargewicht (M_{w}) liegt bei 5.000 bis 100.000, bevorzugt zwischen 10.000 und 30.000. Solche Polymere sind kommerziell beispielsweise unter den Handelsnamen Eastoflex ® (Eastman) oder Vestoplast ® (Hüls) erhältlich.

### Hydrophiler Füllstoff:

In der genannten Polymermatrix unlösliche, hydrophile Partikel auf der Basis von Cellulose. Bevorzugt ist eine mittlere Partikelgröße von kleiner gleich 100 µm mit einer möglichst gleichförmigen Oberfläche. Solche Materialien sind zum Beispiel unter den Handelsnamen Avicel (FMC) und Elcema (Degussa-Hüls) kommerziell erhältlich.

In einer bevorzugten Ausführungsform der Erfindung enthält die Matrix 0,1 bis 20 Gew.-%, bevorzugt 2 bis 10 Gew.-%, des hyperämisierenden Wirkstoffes, insbesondere natürliche Wirkstoffe des Cayennepfeffers, synthetische Wirkstoffe wie Nonivamid, Nicotinsäurederivate, bevorzugt Benzylnicotinat oder Propylnicotinat.

### Beispielhaft seien Capsaicin

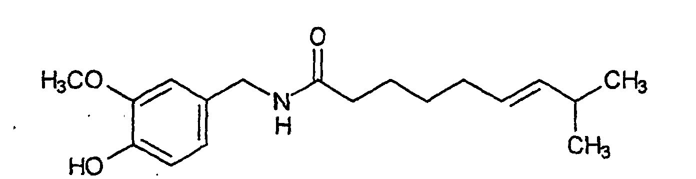

### [8-Methyl-trans-6-nonensäure-(4-hydroxy-3- methoxybenzylamid)]

### Nonivamid

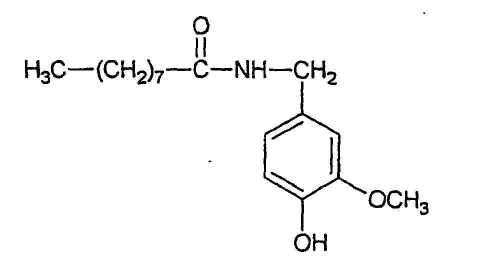

### Nicotinsäurebenzylester

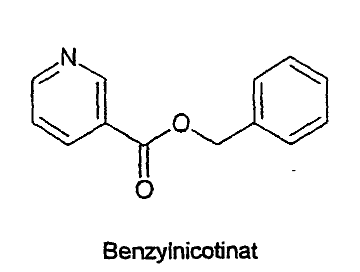

genannt.

Bevorzugt erfolgt die Herstellung der wirkstoffhaltigen Matrix in einem Verfahren, bei dem alle Komponenten der haftklebrigen Matrix unter Verzicht auf den Zusatz von Lösungsmittel in der Schmelze homogenisiert werden.
Besonders bevorzugt werden alle Komponenten in einem kontinuierlichen oder diskontinuierlichen Prozeß bei einer Temperatur unterhalb von 100 °C verarbeitet.

Die Matrix zeichnet sich aus durch hervorragende Hafteigenschaften auf der Haut, durch eine leichte und schmerzfreie Wiederablösbarkeit, sowie vor allem durch sein äußerst geringes Potential, Hautreizungen hervorzurufen. Der Herstellungsprozess verläuft unter vollständigem Verzicht von Lösungsmitteln.
Gegebenfalls kann die offene, auf die Haut zu applizierende Klebseite mit einer wiederablösbaren, abdeckenden Schutzschicht eingedeckt sein.

Bei der Auswahl der Füllstoffe wurde überraschenderweise gefunden, daß sich insbesondere Füllstoffe auf der Basis von Cellulose eignen, die eine isotrope Gestalt besitzen und bei Kontakt mit Wasser nicht zum Quellen neigen. Dabei sind besonders Füllstoffe mit einer Partikelgröße von kleiner gleich 100 µm geeignet.
Der Einsatz hydrophiler Füllstoffe in einer unpolaren Matrix ist in der Literatur bekannt Explizit für den Einsatz in transdermal therapeutischen Systemen werden Sie in EP 0 186 019 beschrieben. Hier allerdings lediglich bis zu einer Konzentration von 3 bis 30 Gew.-%, ohne daß Details zu diesen Füllstoffen erwähnt werden. Die Erfahrung zeigt, daß Systeme mit einem Füllstoffgehalt von über 30 Gew.-% deutlich an Klebrigkeit verlieren und hart und spröde werden. Dadurch verlieren sie die grundlegende Anforderung an ein transdermal therapeutisches System.
Im Rahmen der vorliegenden Erfindung konnte jedoch gezeigt werden, das Füllstoffe auf der Basis von mikrokristalliner oder amorpher Cellulose dann in wesentlich höheren Konzentrationen ohne negative Beeinflussung der klebtechnischen Eigenschaften eingesetzt werden können, wenn sie eine isotrope Gestalt mit einer Partikelgröße von nicht größer als 100 µm besitzen. Höhere Gehalte an Füllstoffen sind zur Verbesserung der Trageeigenschaften insbesondere bei lange andauernder und wiederholter Anwendung wünschenswert.

Die aus den Materialien zur Stoma-Versorgung bekannten hydrophilen Füllstoffe sind in die erfindungsgemäße Matrix integriert, was zur Unterstützung der Hautverträglichkeit dient.

Das Ziel - die topische Applikation von Arzneistoffen aus der Gruppe der - hyperämisierenden Wirkstoffe, die gegebenenfalls unter Einsatz sehr hautverträglicher Additive unterstützt werden - kann im Rahmen der vorliegenden Erfindung durch den Zusatz permeationsfördemden Hilfsstoffe wie zum Beispiel Fettsäureester unterstützt werden.

Überraschenderweise konnten die genannten Anforderungen insbesondere durch ein System realisiert werden, das neben Polyisobutylenen amorphe Poly-α-olefine in Kombination mit amorpher oder mikrokristalliner Cellulose enthält Dieses einfache System enthält als Polymerbasis ausschließlich synthetische Inhaltsstoffe, deren Qualität sehr gut kontrollierbar ist. Dadurch können allergene Reaktionen weitgehend ausgeschlossen werden. Der vollständige Verzicht auf schlecht definierte Inhaltsstoffe wie zum Beispiel Naturkautschuk oder Klebharze führt dadurch zu besonders hautfreundlichen Matrizes. Die klebtechnischen Eigenschaften der erfindungsgemäßen Formulierung sind darüber hinaus sehr gut einstellbar. Des weiteren kann auf eine zusätzliche Additivierung des Systems zur Stabilisierung verzichtet werden.
Wie bereits angedeutet, können besonders hautfreundliche Systeme zur topischen Applikation hyperämisierender Wirkstoffe auf der Basis von Polyisobutylenen unter Einsatz amorpher Poly-α-olefine sowie Cellulose-Partikel als Füllstoff realisiert werden.

Der besondere Vorteil dieser Rohstoffbasis besteht in der Verwendung ausschließlich voll gesättigter synthetischer Elastomere. Diese sind sehr gut definiert und charakterisiert, wodurch die Verunreinigung mit allergenen Begleitstoffen ausgeschlossen werden kann. Durch den hohen Sättigungsgrad sind diese eingesetzten Polymere sehr oxidationsstabil. Deshalb kann auf eine zusätzliche Additivierung mit Antoxidantien und sonstigen Stabilisatoren verzichtet werden. Eine solche Additivierung, wie sie beim Einsatz von Naturkautschuk beziehungsweise ungesättigter Synthesekautschuke notwendig ist, birgt aufgrund der chemischen Struktur der gebräuchlichen Additive immer die Gefahr einer Hautunverträglichkeit. Zusätzlich stellt sie einen zusätzlichen Kostenaufwand dar.
Des weiteren besitzen alle eingesetzten Elastomere je nach Höhe des Molekulargewichtes eine inhärente Klebrigkeit. Dadurch kann zusätzlich auf den Einsatz von Klebharzen verzichtet werden. Klebharze sind häufig auf der Basis von Kolophonium hergestellte Stoffgemische, die sehr schlecht definiert sind. Eine einheitliche Strukturformel kann in den seltensten Fällen angegeben werden. Dies erschwert den Einsatz von Klebharzen als Rohstoff in zulassungspflichtigen Arzneimitteln, wie im vorliegen Fall der transdermal therapeutischen Systeme.
Aufgrund des molekulargewichtsabhängigen Adhäsionsvermögens auf Haut sowohl der Polyisobutylene als auch der amorphen Poly-α-olefine lassen sich die klebtechnischen Eigenschaften des erfindungsgemäßen Systems in einem sehr weiten Bereich einstellen, ohne die Chemie der Basiskomponenten verändern zu müssen. Häufig genügt eine geringe Variation der prozentualen Verhältnisse der Grundkomponenten, um gewünschte Produkteigenschaften zu erhalten.
Dieser Aspekt ist vor allem innerhalb der Arzneimittelentwicklung sehr wichtig. Die sorgfältige Auswahl unbedenklicher und gut hautverträglicher Rohstoffe führt zu einem hohen Kosten- und Zeitaufwand. Es ist daher wünschenswert, Produkteigenschaften gezielt über Variation der prozentualen Zusammensetzung der bekannten Rohstoffe einstellen zu können. Der zeitaufwendige Austausch eines kompletten Rohstoffes wird dadurch vermieden.

Überraschenderweise weist zudem das erfindungsgemäße System (siehe Beispiel 19; Wirkstoffgehalt: 0,125 Gew.-%) neben diesen Gesichtspunkten eine Wirkstofffreisetzung von insgesamt 3,71 µg/(cm² 24 h) auf, das damit dem Vierfachen der Freisetzung des im Markt befindlichen Produkts ABC Wärmepflaster® (Wirkstoffgehalt: 0,137 Gew.-%; Freisetzung: 0,91 µg/(cm² 24 h) entspricht.

### Beispiele

### Beispiele 1 bis 17

Zur Überprüfung des Einflusses verschiedener Inhaltsstoffe der haftklebrigen Matrix hinsichtlich des Haftungsvermögens auf Haut wurden 17 Vergleichsrezepturen im Rahmen eines statistischen Versuchsplans hergestellt.
Das Klebeverhalten der Massesysteme auf Haut wurde in einem Tragetest durch 6 freiwillige Probanden geprüft. Aus diesem Grund wurde zunächst auf die Einarbeitung des Arzneistoffes verzichtet. Bewertet wurden die Muster innerhalb eines Schulnotensystems auf einer Skala von 1 bis 6, wobei 1 die beste Bewertung darstellt, 6 die schlechteste Bewertung.

Die Labormuster wurden nach der folgenden allgemeinen Vorgehensweise hergestellt:

In einem mit Duplex-Schaufeln ausgestatteten Laborkneter wurde bei einer Temperatur von 100 °C die angegebene Menge Vistanex MM L80 vorgelegt und eine Stunde lang geknetet bis das Material krümelig ist. Anschließend wurden nacheinander die angegebenen Mengen Vistanex LM MH, Klebharz und amorphes Poly-α-olefin zugegeben und eine weitere Stunde geknetet, bis das Material homogen ist. Abschließend wurde der Füllstoff in der angegebenen Menge zugegeben und weiterhin eine Stunde geknetet. Das Material wurde nach dem Abkühlen aus dem Kneter entfernt.
Anschließend wurde die Masse zwischen silikonisiertem Papier mit Hilfe einer Heißpresse bei ca. 120 °C auf eine Dicke von 500 µm ausgepreßt. Diese Muster wurden einseitig mit einer Rückenschicht aus Polypropylen laminiert und auf der dieser Schicht abgewandten Seite mit einer silikonisierten Polyester Folie abgedeckt.

Aus diesem Aufbau wurden ca. 2,0 x 6,0 cm² große Muster ausgestanzt, die die Form handelsüblicher Pflasterstrips besitzen.

Die so hergestellten Muster wurden durch die Probanden an der Innenseite der Unterarme aufgeklebt und über einen Zeitraum von 6 h getragen. Bewertet wurden der initiale tack der Muster auf Haut sowie das Haftungsvermögen der Muster über einen Zeitraum von 6 h.

Tabelle 1 enthält die nach dieser Vorgehensweise verarbeiteten Beispielrezepturen 1 bis 17.

**Tabelle 1:**

| Beispielrezepturen 1 - 17 | | | | |
|---|---|---|---|---|
| | PIB I | PIB II | Modifizierendes Agens | Füllstoff |
| Beispiel 1 | 17,0 | 30,0 | 5,0 / I | 48,0 / I |
| Beispiel 2 | 20,0 | 50,0 | 5,0 / I | 25,0 / I |
| Beispiel 3 | 10,0 | 30,0 | 20,0 / II | 40,0 / I |
| Beispiel 4 | 10,0 | 50,0 | 20,0 / III | 20,0 / I |
| Beispiel 5 | 17,0 | 43,0 | 20,0 / III | 20,0 / I |
| Beispiel 6 | 7,0 | 38,0 | 5,0 / IV | 50,0 / I |
| Beispiel 7 | 10,0 | 30,0 | 20,0 / I | 40,0 / II |
| Beispiel 8 | 7,0 | 50,0 | 5,0 / II | 38,0 / II |
| Beispiel 9 | 20,0 | 50,0 | 5,0 / II | 25,0 / II |
| Beispiel 10 | 7,0 | 38,0 | 5,0 / III | 50,0 / II |
| Beispiel 11 | 17,0 | 43,0 | 20,0 / IV | 20,0 / II |
| Beispiel 12 | 20,0 | 30,0 | 20,0 / IV | 30,0 / II |
| Beispiel 13 | 7,0 | 50,0 | 20,0 / I | 23,0 / III |
| Beispiel 14 | 17,0 | 30,0 | 20,0 / II | 33,0 / III |
| Beispiel 15 | 20,0 | 30,0 | 5,0 / III | 45,0 / III |
| Beispiel 16 | 10,0 | 50,0 | 5,0 / IV | 35,0 / III |
| Beispiel 17 | 14,0 | 39,0 | 12,0 / IV | 35,0 / III |

Folgende Rohstoffe wurden verwendet:
- PIB I:: Polyisobutylen, Vistanex MM L80, Exxon Chemical
- PIB II:: Polyisobutylen, Vistanex LM MH, Exxon Chemical

### Modifizierende Inhaltsstoffe:

- I:: Aliphatisch / Aromatisches Kohlenwasserstoffharz, Escorez 2101, Exxon Chemical
- II:: Hydriertes Polycyclopentadienharz, Escorez 5300 I, Exxon Chemical
- III:: Hotmelt Adhesive, Duro Tack H 1540, National Starch
- IV:: amorphes Poly-α-olefin, Eastoflex E 1003, Eastmann

### Füllstoffe:

- I:: Cellulose Fasern, Just Fiber, International Filler of Belgium
- II:: Mikrokristalline Cellulose, Avicel PH 101, FMC
- III:: Kolloidales Silica, HiSil, PPG Industries

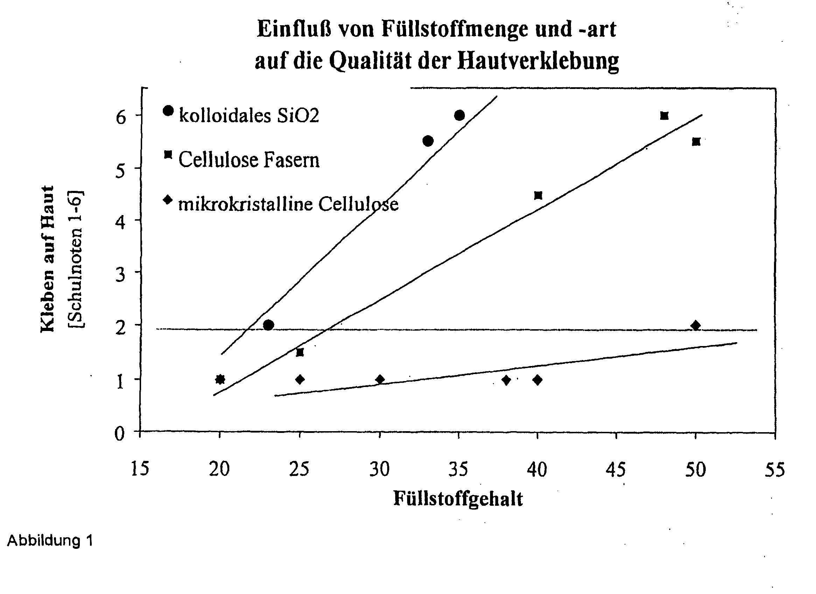

Die klebtechnischen Eigenschaften auf Haut der so hergestellten Muster wurden nach einem Schutnotensystem von 1 bis 6 bewertet Dabei stellt 1 die beste zu vergebende Note dar, 6 die schlechteste zu vergebende Note. Die Ergebnisse dieses Tragetests sind in Tabelle 2 zusammengestellt sowie in Abbildung 1 graphisch dargestellt.

**Tabelle 2:**

| Bewertung des Klebens auf Haut (./.: keine meßbare Adhäsion zu Haut) | | | | | | | | | | | | | | | | | |
|---|---|---|---|---|---|---|---|---|---|---|---|---|---|---|---|---|---|
| Rezeptur | 1 | 2 | 3 | 4 | 5 | 6 | 7 | 8 | 9 | 10 | 11 | 12 | 13 | 14 | 15 | 16 | 17 |
| Bewertung | 6 | 1,5 | 4,5 | 1 | 1 | 5,5 | 1 | 1 | 1 | 2 | 1 | 1 | 2 | 5,5 | ./. | 6 | 6 |

Aus der Abbildung ist deutlich zu erkennen, daß die Art und die Menge des Füllstoffes das Klebeverhalten der Systeme dominieren. Unterhalb einer Einsatzmenge von ca. 20 - 25 Gew.-% zeigen alle Systeme unabhängig vom eingesetzten Füllstoff ein mit mindestens "Gut" (2,0) bewertetes Klebeverhalten. Dies verändert sich drastisch, wenn die Füllstoffmenge auf über 30 Gew.-% angehoben wird:
Oberhalb einer Füllstoffmenge von 30 Gew.-% zeigen solche Systeme ein sehr gutes Klebeverhalten, in denen erfindungsgemäß Cellulose mit einer durchschnittlichen Partikelgröße von 50 µm eingesetzt werden.

### Beispiele 18 bis 20

Zur Überprüfung der Wirkstofffreisetzung eines erfindungsgemäßen Systems werden drei Labormuster basierend auf folgender Placebo-Rezeptur nach der allgemeinen Herstellbeschreibung präpariert.

| | |
|---|---|
| Vistanex LM MH: | 32,0 Gew.-% |
| Vistanex MM L80: | 16,1 Gew.-% |
| Eastoflex PLS E1003D | 16,9 Gew.-% |
| Avicel PH 101: | 35,0 Gew.-% |

Die Wirkstoffdotierung erfolgt beispielhaft mit natürlichem Capsicumextrakt in drei unterschiedlichen Konzentrationen, bezogen auf die Gesamtrezeptur:

| | |
|---|---|
| Beispiel 18 | 0,250 Gew.-% |
| Beispiel 19 | 0,125 Gew.-% |
| Beispiel 20 | 0,050 Gew.-% |

Aus den Labormustem werden jeweils 5 Proben mit einem Durchmesser von 1,80 cm ausgestanzt und auf ihr Freisetzungsverhalten auf Schweinehaut untersucht.
Hierzu wird eine Probe auf ein Stück Schweinehaut appliziert, das auf ein Freisetzungsgefäß nach Franz gelegt wird. Das Freisetzungsgefäß ist mit einer Rezeptorphase gefüllt, die auf konstante 35,5 °C temperiert ist und ständig gerührt wird. Nach 24 h wird der Gehalt an Capsaicinoiden in der Haut und der Rezeptorphase quantitativ bestimmt.

| Ergebnisse: | | | |
|---|---|---|---|
| **Gehalt an Capsaicinoiden** **[µg/cm**^{**2**}**]** | **Beispiel 18** | **Beispiel 19** | **Beispiel 20** |
| Haut | 4,37 | 3,48 | 1,53 |
| Rezeptorphase | 0,21 | 0,23 | 0,04 |
| Summe | 4,58 | 3,71 | 1,57 |

## Patentansprüche

1. Wirkstoffhaltiges Matrixpflaster zur kontrollierten Abgabe von mindestens einem hyperämisierenden Wirkstoff an die Haut, bestehend aus einer flexiblen Deckschicht und einer wirkstoffhaltigen, wasserunlöslichen haftklebrigen Matrix, **dadurch gekennzeichnet, daß** die haftklebrige Matrix frei von Mineralölen und Klebharzen ist und aufgebaut ist aus
a) synthetischen Gerüstpolymeren auf der Basis von Polyisobutylen zu 25 bis 90 Gew.-%,
b) amorphem Poly-α-olefin zu 5 bis 40 Gew.-%,
c) aus einem unlöslichen, hydrophilen Füllstoff mit einer durchschnittlichen Korngröße von weniger als 100 µm zu 10 bis 60 Gew.-% und
d) dem hyperämisierenden Wirkstoff zu 0,001 bis 20 Gew.-%.

2. Wirkstoffhaltiges Matrixpflaster nach Anspruch 1, **dadurch gekennzeichnet, daß** die Matrix Poly-α-olefine in einer Konzentration von 5 bis 20 Gew.-% enthält.

3. Wirkstoffhaltiges Matrixpflaster nach Ansprüchen 1 und 2, **dadurch gekennzeichnet, daß** sich das Polyisobutylen zusammensetzt aus
hochmolekularem PIB zu 5 bis 30 Gew.-% und
niedermolekularem PIB zu 20 bis 60 Gew.-%.

4. Wirkstoffhaltiges Matrixpflaster nach Ansprüchen 1 bis 3, **dadurch gekennzeichnet, daß** der Füllstoff auf Cellulose sowie seinen Derivaten basiert.

5. Wirkstoffhaltiges Matrixpflaster nach Ansprüchen 1 bis 4, **dadurch gekennzeichnet, daß** die Matrix bevorzugt einen hydrophilen Füllstoff auf der Basis von Cellulose sowie seinen Derivaten enthält, deren mittlere Korngröße im Bereich von 20 bis 60 µm beträgt.

6. Wirkstoffhaltiges Matrixpflaster nach Ansprüchen 1 bis 5, **dadurch gekennzeichnet, daß** die Matrix 0,1 bis 20 Gew.-%, bevorzugt 2 bis 10 Gew.-%, eines hyperämisierenden Wirkstoffes enthält, insbesondere natürliche Wirkstoffe des Cayennepfeffers, synthetische Wirkstoffe wie Nonivamid, Nicotinsäurederivate, bevorzugt Benzylnicotinat oder Propylnicotinat.

7. Wirkstoffhaltiges Matrixpflaster nach Ansprüchen 1 bis 6, **dadurch gekennzeichnet, daß** der Matrix permeationsfördemde Inhaltsstoffe im Konzentrationsbereich von 1,0 bis 30 Gew.-%, bevorzugt 10 bis 25 Gew.-% zugesetzt werden.

8. Verfahren zur Herstellung eines wirkstoffhaltigen Matrixpflasters nach Ansprüchen 1 bis 7, **dadurch gekennzeichnet, daß** alle Komponenten der haftklebrigen Matrix unter Verzicht auf den Zusatz von Lösungsmittel in der Schmelze homogenisiert werden.

9. Verfahren zur Herstellung eines wirkstoffhaltigen Matrixpflasters nach Anspruch 8, **dadurch gekennzeichnet, daß** alle Komponenten in einem kontinuierlichen oder diskontinuienichen Prozeß bei einer Temperatur unterhalb von 100 °C verarbeitet werden.

## Claims

1. Active substance matrix patch for controlled delivery of at least one hyperemic active substance to the skin, comprising a flexible cover layer and a water insoluble, pressure sensitively adhesive active substance matrix, **characterized in that** the adhesive matrix is free from mineral oils and tackifier resins and is composed of
a) from 25 to 90% by weight of synthetic framework polymers based on polyisobutylene,
b) from 5 to 40% by weight of amorphous poly-α-olefin,
c) from 10 to 60% by weight of an insoluble hydrophilic filler having an average particle size of less than 100 µm, and
d) from 0.001 to 20% by weight of the hyperemic active substance.

2. Patch according to Claim 1, **characterized in that** the matrix comprises poly-α-olefins in a concentration of from 5 to 20% by weight.

3. Patch according to either of Claims 1 and 2, **characterized in that** the polyisobutylene is composed of
from 5 to 30% by weight of high molecular mass PIB and
from 20 to 60% by weight of low molecular mass PIB.

4. Patch according to any of Claims 1 to 3, **characterized in that** the filler is based on cellulose and its derivatives.

5. Patch according to any of Claims 1 to 4, **characterized in that** the matrix preferably comprises a hydrophilic filler based on cellulose and its derivatives whose average particle size is in the range from 20 to 60 µm.

6. Patch according to any of Claims 1 to 5, **characterized in that** the matrix contains from 0.1 to 20% by weight, preferably from 2 to 10% by weight, of a hyperemic active substance, especially natural active substances from cayenne pepper, synthetic active substances such as nonivamide, or nicotinic acid derivatives, preferably benzyl nicotinate or propyl nicotinate.

7. Patch according to any of Claims 1 to 6, **characterized in that** permeation enhancing ingredients in the concentration range from 1.0 to 30% by weight, preferably from 10 to 25% by weight, are added to the matrix.

8. Process for producing an active substance matrix patch according to any of Claims 1 to 7, **characterized in that** all components of the pressure sensitively adhesive matrix are homogenized in the melt without any addition of solvent.

9. Process according to Claim 8, **characterized in that** all components are processed in a continuous or batchwise process at a temperature below 100°C.

## Revendications

1. Dispositif transdermique matriciel contenant une substance active pour la libération programmée d'au moins une substance active hyperhémiante sur la peau, consistant en une couche de recouvrement flexible et une matrice autoadhésive insoluble dans l'eau, contenant une substance active, **caractérisé en ce que** la matrice autoadhésive est exempte d'huiles minérales et de résines adhésives et est constituée
a) de polymères synthétiques de charpente, à base de polyisobutylène à raison de 25 à 90 % en poids ;
b) d'une poly-α-oléfine amorphe à raison de 5 à 40 % en poids,
c) d'une charge insoluble hydrophile ayant une taille moyenne de particule de moins de 100 µm, à raison de 10 à 60 % en poids et
d) de la substance active hyperhémiante, à raison de 0,001 à 20 % en poids.

2. Dispositif transdermique matriciel contenant une substance active selon la revendication 1, **caractérisé en ce que** la matrice contient des poly-α-oléfines à une concentration de 5 à 20 % en poids.

3. Dispositif transdermique matriciel contenant une substance active selon les revendications 1 et 2, **caractérisé en ce que** le polyisobutylène se compose de
PIB de masse moléculaire élevée, à raison de 5 à 30 % en poids et de
PIB de faible masse moléculaire, à raison de 20 à 60 % en poids.

4. Dispositif transdermique matriciel contenant une substance active selon les revendications 1 à 3, **caractérisé en ce que** la charge est à base de cellulose ainsi que de ses dérivés.

5. Dispositif transdermique matriciel contenant une substance active selon les revendications 1 à 4, **caractérisé en ce que** la matrice contient de préférence une charge hydrophile à base de cellulose ainsi que de ses dérivés, dont la taille moyenne de particule se situe dans la plage allant de 20 à 60 µm.

6. Dispositif transdermique matriciel contenant une substance active selon les revendications 1 à 5, **caractérisé en ce que** la matrice contient de 0,1 à 20 % en poids, de préférence de 2 à 10 % en poids, d'une substance active hyperhémiante, en particulier des substances actives naturelles du poivre de Cayenne, des substances actives de synthèse telles que le novinamide, des dérivés d'acide nicotinique, de préférence le nicotinate de benzyle ou le nicotinate de propyle.

7. Dispositif transdermique matriciel contenant une substance active selon les revendications 1 à 6, **caractérisé en ce qu'**on ajoute à la matrice des composants facilitant la perméation, dans la plage de concentrations allant de 1,0 à 30 % en poids, de préférence de 10 à 25 % en poids.

8. Procédé pour la fabrication d'un dispositif transdermique matriciel contenant une substance active selon les revendications 1 à 7, **caractérisé en ce que** tous les composants de la matrice autoadhésive sont homogénéisés dans la masse fondue, en l'absence d'addition de solvants.

9. Procédé pour la fabrication d'un dispositif transdermique matriciel contenant une substance active selon la revendication 8, **caractérisé en ce que** tous les composants sont traités dans un processus continu ou discontinu, à une température inférieure à 100°C.
